# EUROPÄISCHE PATENTSCHRIFT

(11) **EP 0 304 004 B1**
(45) Veröffentlichungstag und Bekanntmachung des Hinweises auf die Patenterteilung: **03.11.1993**
(21) Anmeldenummer: 88113224.5
(22) Anmeldetag: 16.08.1988
(51) Int. Cl.: C07D 473/02

(54) **Verfahren zur Herstellung von Natriumpurinen**
Process for the production of sodium purines
Procédé pour la préparation de purines de sodium

(30) Priorität: 18.08.1987 DE 3727508
(43) Veröffentlichungstag der Anmeldung: 22.02.1989
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Christmann, Albrecht, Dr., D-6507 Ingelheim am Rhein (DE); Ramert, Reiner, Dr., D-6531 Weiler b. Bingen (DE)

(56) Entgegenhaltungen:
- GB-A- 1 066 325
- HETEROCYCLIC COMPOUNDS, Band 8, Seiten 20-218, 1967, J. Wiley & Sons Inc.; R.C. ELDERFIELD: "Tetrazoles, tetrazines, and purines and related ring system"

## Beschreibung

Die Erfindung betrifft ein neues Verfahren zur Herstellung von Natriumpurinen, insbesondere von Dinatrium-2-amino-1,9-dihydro-purin-6-on bzw. auch als Dinatriumsalz des 2-Amino-1,7- dihydropurin-6-ons bezeichnet und Natrium-1,3-dihydro-7H-purin-2,6-dion.

Dinatrium-2-amino-1,9-dihydro-purin-6-on besitzt einerseits selbst pharmakologische Eigenschaften, kann andererseits aber auch als Ausgangsverbindung zur Herstellung von pharmakologisch aktiven Derivaten eingesetzt werden. Natrium-1,3-dihydro-7H-purin-2,6-dion kann ebenfalls zur Synthese weiterer Pharmazeutika eingesetzt werden. Aus dem Stand der Technik sind verschiedene Verfahren zur Herstellung von Purinen bekannt, so z.B. aus der britischen Patentschrift 1 066 325 wie auch aus R. C. ELDERFIELD "Heterocyclic compounds", Band 8: "Tetrazoles, Tetrazines, and Purines and Related Ring Systems", 1967; JOHN WILEY & SONS, INC., New York - London - Sydney, Seiten 208 - 218.

Nachteil dieser Verfahren ist, daß bei Synthesen in technischem Maßstab das Purin - insbesondere Guanin - in sehr feinkristalliner Form anfällt, das nicht durch einfache Methoden, z.B. abschleudern isoliert werden kann.

Es ist die Aufgabe der vorliegenden Erfindung ein Verfahren zur Herstellung von Natriumpurinen zur Verfügung zu stellen, das in einem "Eintopfverfahren" eine Reaktionsführung in technischem Maßstab und eine Isolierung de : Produkts in hoher Reinheit und hohen Ausbeuten ermöglicht.

Die Aufgabe wird erfindungsgemäß dadurch gelöst, daß man in einer Eintopfreaktion eine Suspension bestehend aus dem entsprechenden 4-Amino-pyrimidinderivat und Natriumnitrit in Formamid mit Ameisensäure versetzt, anschließend reduziert, das gebildete 4-Amino-5-formylamino-pyrimidinderivat dann auf 140° bis 180°C erhitzt und nach erfolgter Ringschlußreaktion durch Zugabe von Natronlauge in das entsprechende Natrium- oder Dinatriumsalz überführt, das als Kristallisat abgetrennt wird.

Einzelheiten des erfindungsgemäßen Verfahrens werden nachfolgend am Beispiel zur Herstellung von Dinatrium-2-amino-1,9-dihydro-purin-6-on erläutert. In einer "Eintopfreaktion" wird eine Suspension bestehend aus 2,4-Diamino-6-oxo-dihydropyrimidin-Monohydrat und Natriumnitrit in Formamid unter Kühlung mit Ameisensäure versetzt. Anstelle des Monohydrats eignet sich für die Umsetzung ebenfalls wasserfreies 2,4-Diamino-6-oxo-dihydropyrimidin (DAHP) oder wasserhaltiges DAHP mit einem Wasseranteil bis zu 30 %. Das molare Verhältnis des Pyrimidins zu Natriumnitrit kann ohne Ausbeuteverluste im Bereich von 1 zu 1 bis 1 zu 1,6 variiert werden. Das Reaktionsgemisch sollte so gekühlt werden, daß die Reaktionstemperatur im Bereich zwischen 10 und 100°C, bevorzugt 20-35°C, besonders bevorzugt zwischen 20-25°C liegt.

Die so erhaltene hellrote Suspension des gebildeten 2,4-Diamino-5-nitroso-6-oxo-dihydropyrimidins wird auf 110 bis 125°C, bevorzugt 110°C, erhitzt und portionsweise mit Natriumdithionit versetzt. Das Gewichtsverhältnis von DAHP zu Natriumdithionit kann in einem weiten Bereich variiert werden, wobei ein möglichst geringer Anteil an Reduktionsmittel angestrebt wird. Eine untere Grenze ist ein Gewichtsverhältnis von DAHP zu Natriumdithionit von 170:1; höhere Mengen an Natriumdithionit sind selbstverständlich möglich, hierbei wird bei der Reaktion jedoch ein höherer Anteil Schwefeldioxid und Schwefelwasserstoff frei.
Anstelle von Natriumdithionit können auch andere Reduktionsmittel eingesetzt werden, wie z. B. Natriumpyrosulfit, Natriumsulfit, Rongalit, Natriumhydrogensulfid, Natriumpolysulfide u.a. Die Zugabe des Reduktionsmittels erfolgt entweder als Festsubstanz oder technisch vorteilhaft in Form einer Lösung. Die Reaktion verläuft exotherm und unter Gasentwicklung. Ein weiterer Zusatz von Reduktionsmittel sollte immer dann erfolgen, wenn die Gasentwicklung bzw. Wärmeentwicklung nachgelassen hat. Nach Ablauf der Reaktion wird die nunmehr hellgelbe Suspension von 2,4-Diamino-5-formamino-6-oxo-dihydropyrimidin, das gegebenenfalls durch Kühlung auf 0°C und Filtration in ca. 96 %iger Ausbeute isoliert werden kann, auf ca. 140 bis 180°C, bevorzugt 175 - 180°C, erhitzt. Die Temperaturabhangigkeit der Cyclisierungsreaktion entspricht einer Arrhenius-Gleichung. Nach ca.1 bis 3 Stunden (bei 175°C) läßt man das Reaktionsgemisch auf ca. 110°C abkühlen und destilliert unter vermindertem Druck das als Lösungsmittel eingesetzte Formamid ab. Der Rückstand wird zuerst mit etwa dem 3- bis 5-fachen Volumen Wasser versetzt, abgekühlt und dann mit dem 2-bis 3-fachen Volumen Natronlauge in Form einer 50 %igen Lösung versetzt, wobei sich die Reaktionslösung dunkelbraun färbt. Durch die Mischungs- bzw. Neutralisationswärme erhöht sich die Temperatur auf ca. 40°C. Ein höherer Anstieg der Temperatur sollte vermieden werden, um die Verseifung von nicht abdestilliertem Formamid möglichst zu vermeiden.

Nach erfolgter Zugabe der Natronlauge läßt man das gebildete Natriumsalz bei einer Temperatur zwischen +5 und -2°C auskristallisieren. Anschließend wird das gebildete Kristallisat abfiltriert und ein- oder mehrfach mit 20 %iger Natronlauge gewaschen. Nach üblicher Trockung erhält man das gewünschte Dinatrium-2-amino-1,9-dihydro-purin-6-on in hoher Ausbeute. Das so erhaltene Produkt kann ohne weitere Reinigungsoperationen für weitere Synthesen, insbesonders für Pharmazeutika, eingesetzt werden.

Andere Purinderivate können, wie zuvor für das Dinatrium-2-amino-1,9-dihydro-purin-6-on beschrieben, aus den entsprechend substituierten Pyrimidinderivaten hergestellt werden.
Die nachfolgenden Beispiele sollen das erfindungsgemäße Verfahren näher erläutern.

### Beispiel 1:

### Dinatrium-2-amino-1,9-dihydropurin-6-on (Dinatriumsalz des 2-Amino-1,7-dihydropurin-6-ons)

36 g 2,4-Diamino-6-oxo-dihydropyrimidin-Monohydrat (250 mmol) und 27,5 g (400 mmol) Natriumnitrit werden in 300 ml Formamid suspendiert und unter Kühlung bei ca. 20-25°C mit 72,5 ml Ameisensäure im Verlauf von ca. 30 Minuten versetzt. Anschließend erhitzt man die hellrote Suspension des entstandenen 2,4-Diamino-5-nitroso-6-oxo-dihydropyrimidins auf ca. 110°C und trägt im Verlauf von ca. 30 Minuten insgesamt 0,25 g Natriumdithionit in Portionen zu ca. 25 mg ein. Nach Ablauf der Reduktion wird die nunmehr hellgelbe Suspension* von 2,4-Diamino-5-formamino-6-oxo-dihydropyrimidin unter Abdestillieren flüchtiger Anteile auf 175 bis 180°C erhitzt und ca. 3 Stunden bei dieser Temperatur gehalten. Nach Abkühlen auf ca. 110°C Sumpftemperatur werden ca. 225 ml Formamid im Wasserstrahlvakuum abdestilliert. Der Destillationssumpf wird mit 250 ml Wasser versetzt, auf ca. 25°C abgekühlt und unter weiterer Kühlung werden 118 ml ca. 50%iger Natronlauge zugegeben, worauf eine dunkelbraune Lösung entsteht. Diese wird auf 0° bis + 3°C abgekühlt und zur Vervollständigung der Kristallisation noch ca. 2 h bei dieser Temperatur gerührt. Die Kristalle werden abgesaugt, mit ca. 90 ml ca. 20%iger Natronlauge gewaschen und im Vakuum bei ca. 80°C getrocknet. Man erhält ca. 55 g eines Salzes, das nach Elementaranalyse und Wasserbestimmung 41,0 g der Titelverbindung entspricht (Ausbeute 84 % d. Th.).
* Kühlt man die erhaltene Suspension nach Ablauf der Reduktion auf 0°C ab und isoliert den Niederschlag, so erhält man ca. 96% d.Th. des 2,4-Diamino-5-formylamino-6-oxo-dihydropyrimidins.

### Beispiel 2:

### Herstellung von Dinatrium-2-amino-1,7-dihydropurin-6-on im technischen Maßstab

28,8 kg 2,4-Diamino-6-oxo-dihydropyrimidinmonohydrat (200 mol) und 16,7 kg (240 mol) Natriumnitrit werden in 240 l Formamid suspendiert und unter Kühlung bei ca. 20-25°C im Verlauf von ca. 60 Minuten mit 70 l Ameisensäure versetzt. Anschließend erhitzt man die hellrote Suspension des entstandenen 2,4-Diamino-5-nitroso-6-oxo-dihydropyrimidins auf ca. 110°C und tropft im Verlauf von ca. 90 Minuten insgesamt 100 g Natriumdithionit, gelöst in 1 l Wasser und 5 l Formamid, zu. Nach Ablauf der Reduktion wird die nunmehr hellgelbe Suspension von 2,4-Diamino-5-formylamino-6-oxo-dihydropyrimidin unter Abdestillieren flüchtiger Anteile auf 150 bis 155°C erhitzt und ca. 16 Stunden bei dieser Temperatur gehalten. Nach Abkühlen auf ca. 110°C Sumpftemperatur werden ca. 200 l Formamid im Dampfstrahlvakuum abdestilliert. Der Destillationssumpf wird mit 200 l Wasser versetzt, auf ca. 25°C abgekühlt und unter weiterer Kühlung werden 95 l ca. 50%iger Natronlauge zugegeben, wobei eine dunkelbraune Lösung entsteht. Diese wird auf 0° bis +3°C abgekühlt und zur Vervollständigung der Kristallisation noch ca. 2 Stunden bei dieser Temperatur gerührt. Die abgetrennten Kristalle werden abgeschleudert, mit ca. 140 l ca. 20%iger eiskalter Natronlauge gewaschen und im Vakuum bei ca. 80°C getrocknet. Es werden ca. 56 kg Produkt erhalten, das gemäß der Stickstoffanalyse (20,4 % N) 31,8 kg der Titelverbindung entspricht.

### Beispiel 3:

### Herstellung von Natrium-1,3-dihydro-7H-purin-2,6-dion (Natriumsalz des Xanthins)

31,8 g 6-Aminouracil (250 mmol) und 27,5 g (400 mmol) Natriumnitrit werden in 400 ml Formamid suspendiert und unter Kühlung bei ca. 20-25°C mit 105,3 g 85%iger Ameisensäure im Verlauf von ca. 3 Stunden versetzt. Anschließend erhitzt man die hellrote Suspension des entstandenen 6-Amino-5-nitroso-uracils auf ca. 120°C und trägt im Verlauf von ca. 30 Minuten insgesamt 0,20 g Natriundithionit in Portionen zu ca. 25 mg ein. Nach Ablauf der Reduktion wird die nunmehr hellbraune Suspension von 6-Amino-5-formamino-uracil unter Abdestillieren flüchtiger Anteile auf ca. 150°C erhitzt und ca. 5 Stunden bei dieser Temperatur gehalten. Nach Abkühlen auf ca. 110°C Sumpftemperatur werden ca. 300 ml Formamid im Wasserstrahlvakuum abgestilliert. Der Destillationssumpf wird mit 480 ml Wasser versetzt, auf ca. 25°C abgekühlt und unter weiterer Kühlung werden 13,5 ml ca. 50%iger Natronlauge zugegeben, wobei eine dunkelbraune Lösung entsteht. Diese wird auf ca. 15°C abgekühlt und zur Vervollständigung der Kristallisation noch ca. 2 Stunden bei dieser Temperatur gerührt. Der abgetrennte Niederschlag wird mit ca. 30 ml kaltem Wasser gewaschen und im Vakuum bei ca. 80°C getrocknet. Es werden ca. 66 g eines Salzes enthalten, das gemäß dem Stickstoffgehalt (16,6 %) 34,1 g der Titelverbindung entspricht (Ausbeute 78 % d. Th.).

Durch Lösen dieses Salzes in verdünnter Natronlauge, Behandlung mit Entfärbungskohle, Fällen mit Ameisensäure und Trocknen bei 60°C i.Vak. werden 21,5 g (72 % d. E.) reines 1,3-Dihydro-7H-purin-2,6-dion erhalten.

## Patentansprüche

1. Verfahren zur Herstellung von Natriumpurinen in einer Eintopfreaktion, wobei man eine Suspension bestehend aus dem entsprechenden 4-Amino-pyrimidinderivat und Natriumnitrit in Formamid mit Ameisensäure versetzt, anschließend reduziert, das gebildete 4-Amino-5-formylaminopyrimidinderivat dann auf 140 bis 180°C erhitzt, dadurch gekennzeichnet, daß man nach erfolgter Ringschlußreaktion durch Zugabe von konzentrierter Natronlauge in das entsprechende Natrium- oder Dinatriumsalz überführt, das als Kristallisat abgetrennt wird.

2. Verfahren zur Herstellung von Dinatrium-2-amino-1,9-dihydto-purin-6-on nach Anspruch 1, dadurch gekennzeichnet, daß eine Suspension von 2,4-Diamino-6-oxo-dihydropyrimidin als Ausgangsverbindung eingesetzt wird.

3. Verfahren zur Herstellung von Natrium-1,3-dihydro-7H-purin-2,6-dion nach Anspruch 1, dadurch gekennzeichnet, daß eine Suspension von 6-Aminouracil als Ausgangsverbindung eingesetzt wird.

## Claims

1. Process for preparing sodium purines in a one-pot reaction, in which a suspension consisting of the corresponding 4-amino-pyrimidine derivative and sodium nitrite in formamide is mixed with formic acid, then the mixture is reduced, the 4-amino-5-formylamino-pyrimidine derivative formed is then heated to 140 to 180°C, characterized in that, after the reaction of cyclisation has occurred, it is converted, by the addition of concentrated sodium hydroxide solution, into the corresponding sodium or disodium salt which is separated off in the form of crystals.

2. Process for preparing disodium-2-amino-1,9-dihydropurin-6-one according to claim 1, characterized in that a suspension of 2,4-diamino-6-oxo-dihydropyrimidine is used as the starting compound.

3. Process for preparing sodium-1,3-dihydro-7H-purin-2,6-dione according to claim 1, characterized in that a suspension of 6-aminouracil is used as the starting compound.

## Revendications

1. Procédé de préparation de purines de sodium par une réaction en réacteur unique, dans lequel on additionne d'acide formique une suspension consistant en le dérivé de 4-aminopyrimidine correspondant et en nitrite de sodium dans le formamide, puis on réduit, on chauffe ensuite à une température de 140 à 180°C le dérivé de 4-amino-5-formylaminopyrimidine formé, caractérisé en ce que, la réaction de cyclisation étant terminée, on le convertit, par addition de lessive de soude concentrée, en le sel sodique ou disodique correspondant que l'on sépare sous forme de produit de cristallisation.

2. Procédé de préparation de la 2-amino-1,9-dihydropurine-6-one disodique selon la revendication 1, caractérisé en ce que l'on utilise comme composé de départ une suspension de 2,4-diamino-6-oxo-dihydropyrimidine.

3. Procédé de préparation de la 1,3-dihydro-7H-purine-3,6-dione de sodium selon la revendication 1, caractérisé en ce que l'on utilise comme composé de départ une suspension de 6-amino-uracile.
